(19) **Europäisches Patentamt / European Patent Office / Office européen des brevets**

(11) **EP 3 843 754 B1**

(12) # EUROPEAN PATENT SPECIFICATION

(45) Date of publication and mention
of the grant of the patent:
**07.02.2024 Bulletin 2024/06**

(21) Application number: **19853302.8**

(22) Date of filing: **26.08.2019**

(51) International Patent Classification (IPC):
**A61K 33/04** (2006.01)   **A61K 31/26** (2006.01)
**A61P 35/00** (2006.01)

(52) Cooperative Patent Classification (CPC):
(C-Sets available)
**A61P 35/00; A61K 31/26; A61K 33/04**   (Cont.)

(86) International application number:
**PCT/PL2019/050047**

(87) International publication number:
**WO 2020/046153 (05.03.2020 Gazette 2020/10)**

(54) **CONJUGATES OF SELENIUM AND ISOTHIOCYANATE NANOPARTICLES FOR USE IN THE TREATMENT OF BREAST CANCER**

KONJUGATE VON SELEN UND ISOTHIOCYANAT-NANOPARTIKELN ZUR VERWENDUNG BEI DER BEHANDLUNG VON BRUSTKREBS

CONJUGUÉS DE SÉLÉNIUM ET DE NANOPARTICULES D'ISOTHIOCYANATE DESTINÉS À ÊTRE UTILISÉS DANS LE TRAITEMENT DU CANCER DU SEIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.08.2018 PL 42679118**

(43) Date of publication of application:
**07.07.2021 Bulletin 2021/27**

(73) Proprietors:
- **Narodowy Instytut Leków W Warszawie**
  **00-725 Warszawa (PL)**
- **Uniwersytet Warszawski**
  **00-927 Warszawa (PL)**
- **Warszawski Uniwersytet Medyczny**
  **02-091 Warszawa (PL)**
- **Instytut Medycyny Doswiadczalnej I Klinicznej Im.**
  **M. Mossakowskiego, Pan**
  **02-106 Warszawa (PL)**

(72) Inventors:
- **WIKTORSKA, Katarzyna**
  **02-791 Warszawa (PL)**
- **MIELCZAREK, Lidia**
  **18-500 Kolno (PL)**

- **CHILMONCZYK, Zdzislaw**
  **01-904 Warszawa (PL)**
- **KRUG, Pamela**
  **02-972 Warszawa (PL)**
- **KARASINSKI, Jakub**
  **26-800 Bialobrzegi (PL)**
- **BULSKA, Ewa**
  **03-977 Warszawa (PL)**
- **MAZUR, Maciej**
  **03-141 Warszawa (PL)**
- **KACZYNSKA, Katarzyna**
  **03-140 Warszawa (PL)**

(74) Representative: **JD&P Patent Attorneys**
**Joanna Dargiewicz & Partners**
**Ul. Mysliborska 93A/50**
**03-185 Warszawa (PL)**

(56) References cited:
**CN-A- 102 657 866    CN-A- 106 668 060**
**PL-A1- 402 631**

**(Cont. next page)**

- KUMARI MANISHA ET AL: "Curcumin loading potentiates the chemotherapeutic efficacy of selenium nanoparticles in HCT116 cells and Ehrlich's ascites carcinoma bearing mice", EUROPEAN JOURNAL OF PHARMACEUTICS AND BIOPHARMACEUTICS, ELSEVIER SCIENCE PUBLISHERS B.V., AMSTERDAM, NL, vol. 117, 9 May 2017 (2017-05-09), pages 346-362, XP085093107, ISSN: 0939-6411, DOI: 10.1016/J.EJPB.2017.05.003
- TIN KHOR ET AL: "Dietary Cancer Chemopreventive Agents – Targeting Inflammation and Nrf2 Signaling Pathway", PLANTA MEDICA, vol. 74, no. 13, 30 July 2008 (2008-07-30) , pages 1540-1547, XP055370069, DE ISSN: 0032-0943, DOI: 10.1055/s-0028-1088303
- BRYAN HOLLY K ET AL: "The Nrf2 cell defence pathway: Keap1-dependent and -independent mechanisms of regulation", BIOCHEMICAL PHARMACOLOGY, ELSEVIER, US, vol. 85, no. 6, 5 December 2012 (2012-12-05), pages 705-717, XP028981638, ISSN: 0006-2952, DOI: 10.1016/J.BCP.2012.11.016
- YICHONG WANG ET AL: "Synergy between sulforaphane and selenium in protection against oxidative damage in colonic CCD841 cells", NUTRITION RESEARCH, vol. 35, no. 7, 30 May 2015 (2015-05-30), pages 610-617, XP055697189, AMSTERDAM, NL ISSN: 0271-5317, DOI: 10.1016/j.nutres.2015.05.011
- WANG Y. et al.: "Synergy between sulforaphane and selenium in protection against oxidative damage in colonic CCD841 cells", Nutr Res., vol. 35, no. 7, 30 May 2015 (2015-05-30), - July 2015 (2015-07), pages 610-617, XP055697189,
- J.ZHANG et al.: "Synergy between sulforaphane and selenium in the induction of thioredoxin reductase 1 requires both transcriptional and translational modulation", Carcinogenesis, vol. 24, no. 3, 1 March 2003 (2003-03-01), pages 497-503, XP055697190,

(52) Cooperative Patent Classification (CPC): (Cont.)

C-Sets
A61K 31/26, A61K 2300/00;
A61K 33/04, A61K 2300/00

**Description**

FIELD OF THE INVENTION

The invention relates to conjugates of selenium and isothiocyanate nanoparticles having synergistic anti-tumour properties enhanced compared to the properties of the individual components of the conjugate for use in the treatment of breast cancer, characterized by the absence of estrogen, progesterone and HER-2/Neu receptors.

PRIOR ART

Selenium is an important microelement that has both anti-tumour and chemopreventive effects at various stages of carcinogenesis. However, selenium is also characterised by high systemic toxicity, which limits its clinical use.

Various mechanisms of its action have been proposed, including the effect of antioxidation and detoxification, regulating cell proliferation, enhancing immunological control and of inhibiting tumour cell metastasis and angiogenesis processes [H. Zeng et al.; Selenium as an anticancer nutrient: roles in cell proliferation and tumour cell invasion; International Journal of Nutritional Biochemistry, 2008, (19): 1-7].

The toxicity of selenium depends on its chemical form: the toxicity of organic selenium compounds is lower than that of inorganic selenium compounds. Elementary selenium with an oxidation degree of 0 is insoluble, which results in a very low biological activity level thereof. The use of nanotechnology, however, makes it possible to significantly increase the biological activity of elementary selenium while maintaining its low toxicity. Toxicity studies of selenium nanoparticles [J. Zhang et al.; Elemental Selenium at Nano Size (Nano-Se) as a Potential Chemopreventive Agent with Reduced Risk of Selenium Toxicity: Comparison with Se-methylselenocysteine in Mice; Toxicol. Sci. 2008, (101): 22-31] demonstrated that their toxicity is lower than that of the organic selenium compound Se-methylselenocysteine, so that they could be useful in the treatment of tumour.

Publication Kumari Manisha et al, "Curcumin loading potentiates the chemotherapeutic efficacy of selenium nanoparticles in HCT116 cells and Ehrlich's ascites carcinoma bearing mice", European Journal of Pharmaceutics and Biopharmaceutics vol. 117, (2017), relates to selenium nanoparticles having improved chemotherapeutic efficiency and disclose the preparation of curcumin loaded selenium nanoparticles, the molecules of curcumin are immobilised on the surface of the selenium nanoparticles.

Tin Khor et al, "Dietary Cancer Chemopreventive Agents - Targeting Inflammation and Nrf2 Signaling Pathway", Planta Medica, DE, vol. 74, no. 13, (2008) mentions that curcumin and isothiocyanates are both natural phytochemicals that showed anti-inflamatory and cancer chemopreventive effects in rodent and human trials.

Bryan Holly K et al, "The Nrf2 cell defence pathway: Keap1-dependent and - independent mechanisms of regulation", Biochemical Pharmacology, Elsevier, US, vol. 85, no. 6 (2012), teaches that Nrf2 contributes to diverse cellular functions including differentiation, proliferation, inflammation and lipid synthesis and there is an increasing association of aberrant expression and/or function of Nrf2 with pathologies including cancer, neurodegeneration and cardiovascular disease and mentions that that curcumin and isothiocyanates have similar relevant mechanism with respect to cancer, namely induction of Nrf2.

From the prior art combinations are known of compounds containing selenium and other compounds having anti-tumour properties, which were proved to have the so-called synergistic effect, i.e. the result of their joint action is stronger than the cumulative actions of the individual components would suggest.

The publication [W. Liu et al.; Selenium nanoparticles as a carrier of 5-fluorouracil to achieve anticancer synergism; ACS Nano. 2012, 6(8): 6578-6591] cites an example of the use of selenium nanoparticles modified with 5-fluorouracil. A strong synergy of action between selenium and 5-fluorouracil nanoparticles to A375 malignant melanoma cells was observed for this conjugate.

Isothiocyanates, including natural isothiocyanates such as sulforaphane (SFN), also have strong anti-oxidant and detoxifying properties. Sulforaphane has complex anti-tumour effect at various stages of carcinogenesis. The action of sulforaphane at the onset of carcinogenesis involves eliminating mutagenic and genotoxic factors. During the carcinogenesis process, sulforaphane exerts an anti-proliferative effect and induces apoptosis. At the cancer progression phase, in turn, sulforaphane reduces angiogenesis and metastasis. It also has an anti-bacterial and anti-inflammatory effect [J. Tomczyk et al.; Sulforaphane - a possible agent in prevention and therapy of cancer; PostepyHig. Med. Dosw. 2010, (64): 590-603].

Together with selenium in the form of sodium selenate, sulforaphane has a synergistic anti-oxidant effect on normal colonic CCD841 cells [Y. Wang et al, Synergy between sulforaphane and selenium in protection against oxidative damage in colonic CCD841 cells; Nutr. Res. 2015, 35(7): 610-617] and on liver cancer HepG2 cells [D. Li et al.; Synergy between sulforaphane and selenium in the up-regulation of thioredoxin reductase and protection against hydrogen peroxide-induced cell death in human hepatocytes; Food Chem. 2012, 133(2): 300-307]. Prior art provides no data, however, on the effect of sulforaphane and selenium in the form of selenium nanoparticles.

Prior art provides no data on whether sulforaphane could affect the activity of selenium nanoparticles in tumour and normal cells, thus increasing their selectivity.

The use of nanoparticles as drug carriers is also known from prior art. Drug carriers are intended to increase drug efficacy, i.e. increase its accumulation in tumour tissue, while reducing its toxicity to healthy tissue. Publication US2011010262564 discloses a method for obtaining selenium nanoparticles and determining their cytotoxicity *in vitro*.

Prior art documents do not report any examples of the use of conjugates of selenium nanoparticles with isothiocyanate compounds in order to enhance their effect on tumour cells. Neither are there any reports of antagonistic effect of selenium nanoparticles modified with isothiocyanates on normal cells.

DISCLOSURE OF THE INVENTION

This invention relates to a conjugate of selenium nanoparticles with sulforaphane as isothiocyanate having anti-tumour properties against, breast cancer characterised by the absence of estrogen, progesterone and HER-2/Neu receptors. Unexpectedly, *in vitro* studies have shown that conjugates composed of selenium nanoparticles and sulforaphane as isothiocyanates, have increased anti-tumour activity, especially towards breast tumour cells MCF-7 and MDA-MB-231. Also, this conjugate has reduced cytotoxicity towards normal colon CRL-1790 and breast MCF-10A cells. The studies were conducted using MTT and CVS cytotoxicity assays measuring the ability of a compound to reduce cell viability and number, respectively. The inventors demonstrated that in both tests the conjugate had a synergistic effect of selenium nanoparticles and sulforaphane on tumour cells and an antagonistic effect on normal cells. The prepared conjugate also demonstrated a very high selectivity towards the tumour cells of colon cancer Caco-2, HT-2 and prostate cancer PC-3.

The solution according to the invention relates to a conjugate having anti-tumour properties containing selenium nanoparticles modified with at least one isothiocyanate as a substance enhancing the effect of the selenium nanoparticles for use in the treatment of tumour, wherein the at least one isothiocyanate is sulforaphane immobilised on the surface of selenium nanoparticles, and wherein the tumour is breast cancer characterised by the absence of estrogen, progesterone and HER-2/Neu receptors.

The preferred conjugate for use in the treatment of breast cancer characterised by the absence of estrogen, progesterone and HER-2/Neu receptors the nanoparticles of the conjugate have the size of the diameter in the range $80.2 \pm 18.6$ nm as measured with transmission electron microscopy (TEM).

The description also describes not covered by the subject -matter of claims conjugates having anti-cancer properties containing selenium nanoparticles modified with at least one isothiocyanate as a substance enhancing the effect of the selenium nanoparticles for use in the treatment of tumour, wherein the at least one isothiocyanate is immobilised on the surface of selenium nanoparticles. In such a conjugate, selenium nanoparticles are coated with a compound that enhances their effect, selected from a group of isothiocyanates or mixtures thereof. Preferably, the tumours treated with said conjugate are tumours selected from breast tumour, ovarian tumour, prostate tumour, gastric tumour, thyroid tumour, small-cell lung cancer, liver tumour, squamous cell carcinoma of head and neck, lymphomas, multiple myeloma, Hodgkin's disease, and particularly preferably it is breast tumour. Particularly according to the invention, the conjugate of selenium nanoparticles coated with sulforaphane is for use in the treatment of aggressive form of breast cancer resistant to other therapies, characterised by the lack of estrogen, progesterone and HER-2/Neu receptors.

The description teaches the substances enhancing the effect of selenium nanoparticles, being isothiocyanates immobilised on the surface of selenium nanoparticles, selected from the group comprising sulforaphane, phenethyl isothiocyanate, benzyl isothiocyanate, allyl isothiocyanate, 3-methylindolisothiocyanate and analogues and degradation products thereof, e.g.: 2-oxoheptyl isothiocyanate, alyssin, 2-oxohexyl isothiocyanate, indole-3-carbinol or mixtures thereof..

The described nanoparticles of the conjugate are of size of the diameter in the range from 60 to 120 nm, more preferably, the diameter of nanoparticles of the conjugate is approximately $80 \pm 20$ nm, more preferably approximately 80 nm, most preferably approximately 100 nm.

In order to produce a conjugate of selenium nanoparticles coated with an isothiocyanate compound (with an immobilised isothiocyanate compound on the surface of the selenium nanoparticles), in the first step the freshly prepared ascorbic acid solution is added dropwise into an aqueous solution containing sodium selenate and the selected isothiocyanate (or a mixture of various isothiocyanates). Subsequently, sodium selenate is reduced to elementary selenium in the form of nanoparticles, and isothiocyanate is adsorbed on the surface of nanoparticles. The solution is then dialysed to remove excess sodium selenate and isothiocyanates. This yields stable conjugates of selenium nanoparticles coated with an isothiocyanate compound for use in the treatment of tumour, including breast tumour. The problem of tumours therapy in Poland and in the world remains unresolved, and more effective therapy methods are still being sought. In *in vitro* studies, the presented invention, i.e. the conjugate of selenium nanoparticles and isothiocyanates, showed cytotoxic properties to breast tumour cells superior to those of selenium nanoparticles and isothiocyanate alone.

Preferably, the conjugate prepared is characterised by a particularly high toxicity to tumours that are resistant to hormone therapy. Breast cancer is the most common malignant tumour, accounting for 22% of all new disease cases in women [J.P. Janssens et al.; Rak piersi: bezpośrednie i pośrednie czynniki ryzyka związane z wiekiem i stylem życia; Nowotwory 2009, 59(3):159-167]. In particular, breast tumour resistant to hormone therapy is very aggressive and difficult to treat. It is a triple-receptor negative breast cancer characterised by the lack of estrogen, progesterone and HER-2/Neu receptors [W. D. Foulkes et al.; Triple-negative breast cancer; N. Engl. J. Med. 2010,363:1938-1948]

The conjugate of the invention has reduced toxicity to normal cells, as demonstrated by measuring the survival rate of normal colon CRL-1790 and breast MCF-10A cells *in vitro*.

Administration of the conjugate according to the invention produces a more potent proapoptotic effect, and, consequently, a more potent cytotoxic effect on tumour cells.

The conjugate of the invention can be administered by various routes, including by injection, orally, locally and per rectum.

The dose of the conjugate is determined taking into account the route of administration, the condition requiring treatment or prevention, and other specific circumstances.

BRIEF DESCRIPTION OF THE FIGURES

To be better understood, the invention has been illustrated in embodiments and in the accompanying figures, where:

Fig. 1 is a TEM image of (A) the conjugate of selenium nanoparticles and sulforaphane (B) selenium nanoparticles (200 nm scale).

Fig. 2 is (A) a SEM image of the conjugate of selenium nanoparticles and sulforaphane with a diameter of 100 nm and (B) their elemental composition obtained by EDS analysis.

Fig. 3 shows the XPS spectra of the conjugate selenium nanoparticles-sulforaphane: (A) survey spectrum, (B) selenium 3d detailed spectrum, (C) 2p sulphur and selenium 3p detailed spectrum, (D) 1s nitrogen detailed spectrum.

Fig. 4 presents the cytotoxic effect of selenium nanoparticles administered in combination with sulforaphane (SeNPs+SFN) and of the conjugate of selenium nanoparticles and sulforaphane (SFN-SeNPs) on MDA-MB-231 cell line.

Fig. 5 shows the values of the $IC_{50}$ parameter of the conjugate selenium nanoparticles-sulforaphane relative to cell lines: MDA-MB-231, MCF-7, PC-3, HT-29, Caco-2, MCF-10A and CRL-1790 obtained by two cytotoxicity assays: MTT and CVS.

Fig. 6 presents the cytotoxic effect of selenium nanoparticles (SeNPs), sulforaphane (SFN) and of the conjugate of selenium nanoparticles and sulforaphane (SFN-SeNPs) on the following cell lines: MDA-MB-231, MCF-7, PC-3, HT-29, Caco-2, MCF-10A and CRL-1790 obtained by two cytotoxicity assays: MTT and CVS.

Fig. 7 presents the correlation between the CI value (determining the type of the interaction observed) and the fraction of dead cells (determining the number of dead cells) for the conjugate of selenium nanoparticles and sulforaphane to the (A) MDA-MB-231, (B) MCF-7, (C) CRL-1790 and (D) MCF-10A cell line.

Fig. 8 shows the concentration of selenium in rat organs following the administration of a dose of 100mg/kg of the conjugate of selenium nanoparticles and sulforaphane after 24 hours.

Fig. 9 shows the percentage [%] of cells: A) MDA-MB-231 and B) MCF-10A, in the individual phases of the cell cycle sub-Gi, $G_0/G_1$, S and $G_2/M$. The study was performed 72 hours after addition of sulforaphane (SFN), non-modified selenium nanoparticles (SeNPs), non-modified selenium nanoparticles mixed with sulforaphane (SFN + SeNPs) and selenium nanoparticles modified (on the surface) with sulforaphane (SFN-SeNPs), respectively.

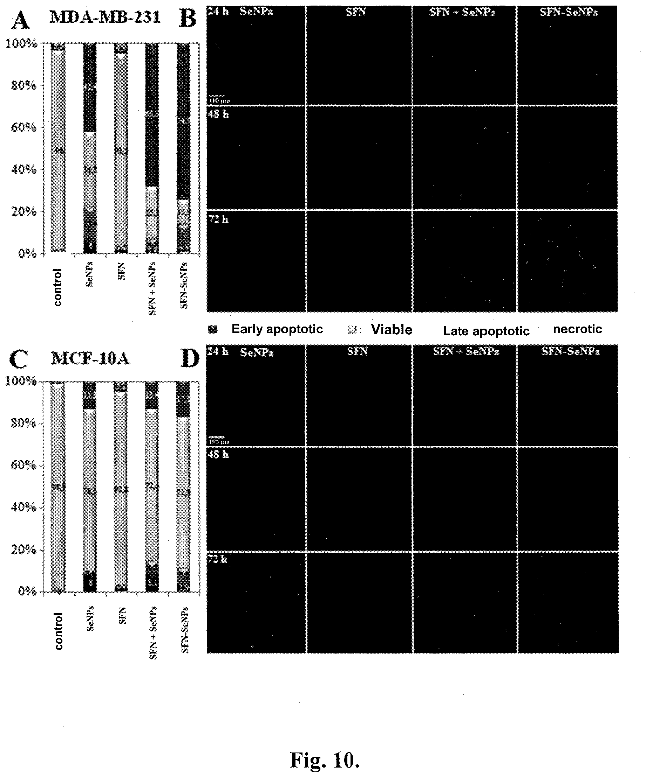

Fig. 10 shows: A) percentage [%] of necrotic, late apoptotic, early apoptotic and viable MDA-MB-231 cells; the study was performed 72 hours after adding, respectively, the following: sulforaphane (SFN), non-modified selenium nanoparticles (SeNPs), non-modified selenium nanoparticles mixed with sulforaphane (SFN + SeNPs) and selenium nanoparticles modified (on the surface) with sulforaphane (SFN-SeNPs); B) images of MDA-MB-231 cells after 24, 48 and 72 hours after adding, respectively, the following: sulforaphane (SFN), non-modified selenium nanoparticles (SeNPs), non-modified selenium nanoparticles mixed with sulforaphane (SFN + SeNPs) and selenium nanoparticles modified (on the surface) with sulforaphane (SFN-SeNPs); the cells were stained with Annexin V FITC (green - apoptotic cells) and propidium iodide (red - dead cells); C) percentage [%] of necrotic, late apoptotic, early apoptotic and viable MCF-10A cells; the study was performed 72 hours after adding, respectively, the following: sulforaphane (SFN), non-modified selenium nanoparticles (SeNPs), non-modified selenium nanoparticles mixed with sulforaphane (SFN + SeNPs) and selenium nanoparticles modified (on the surface) with sulforaphane (SFN-SeNPs); D) images of MCF-10A cells after 24, 48 and 72 hours after adding, respectively, the following: sulforaphane (SFN), non-modified selenium nanoparticles (SeNPs), non-modified selenium nanoparticles mixed with sulforaphane (SFN + SeNPs) and selenium nanoparticles modified (on the surface) with sulforaphane (SFN-SeNPs); the cells were stained with Annexin V FITC (green - apoptotic cells) and propidium iodide (red - dead cells).

**Fig. 11** shows images of MDA-MB-231 cells after 24, 48 and 72 hours after adding the medium (control) and, respectively, the following: sulforaphane (SFN), non-modified selenium nanoparticles (SeNPs), non-modified selenium nanoparticles mixed with sulforaphane (SFN + SeNPs) and selenium nanoparticles modified (on the surface) with sulforaphane (SFN-SeNPs) - the cells were stained with MitoLight: red - polarised mitochondrial membrane, green - depolarized mitochondrial membrane.

**Fig. 12** shows images of MCF-10A cells after 24, 48 and 72 hours after adding the medium (control) and, respectively, the following: sulforaphane (SFN), non-modified selenium nanoparticles (SeNPs), non-modified selenium nanoparticles mixed with sulforaphane (SFN + SeNPs) and selenium nanoparticles modified (on the surface) with sulforaphane (SFN-SeNPs) - the cells were stained with MitoLight: red - polarised mitochondrial membrane, green - depolarized mitochondrial membrane.

The invention is illustrated in greater detail in the embodiments below that do not limit the scope thereof.

## EXAMPLES

In the examples below, unless indicated otherwise, standard materials and *in vitro* biochemical methods were employed, or it was proceeded according to the manufacturer's recommendations for particular materials and methods.

### Example 1 Preparation of the conjugate of selenium nanoparticles and sulforaphane

20 mL of 40 mM sulforaphane solution was added to 20 mL of 5mM sodium selenate solution. Then, under vigorous stirring, 10 mL of freshly prepared solution of ascorbic acid (40mM) was slowly added dropwise. 50 mL of deionised water was then added, and this has been reacted for half an hour. Following the reaction, the sample was dialysed for 24h at room temperature. The selenium concentration was determined using the ICP-MS (Inductively Coupled Plasma - Mass Spectrometry) method.

### Example 2 Determination of the sizes of nanoparticles obtained through microscopic measurements

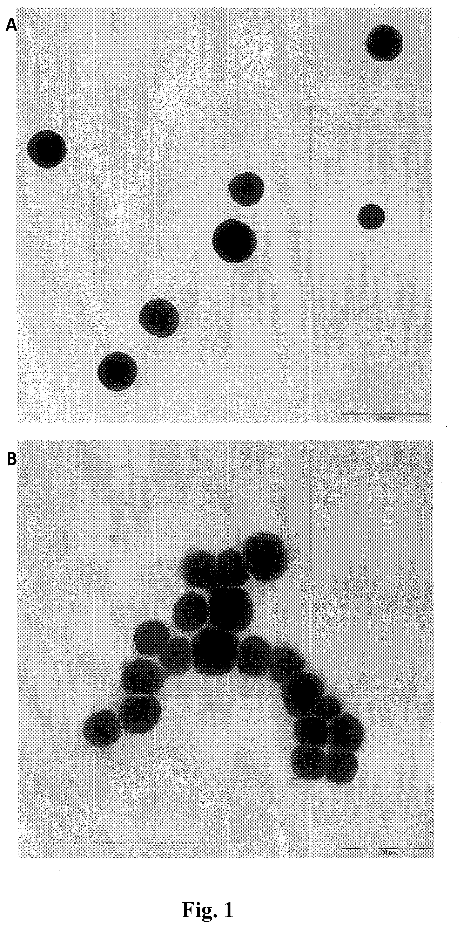

In order to prepare the test samples by way of transmission electron microscopy (TEM), one drop of the prepared sample was added onto the TEM grid and left to dry for 24h. **Fig. 1A** is a representative microscopic image of the conjugate of selenium nanoparticles and sulforaphane. The nanoparticles obtained were homogeneous, stable, dispersed and had a size of 80.2 in diameter + 18.6 nm. The stability and size of the particles is an important parameter due to their medical applicability. Particles in the range of 60 to 120 nm are well distributed in the bloodstream due to their extended circulation time. **Fig. 1B** is an image of non-modified selenium nanoparticles. Selenium nanoparticles form aggregates in the shape of round polyhedrons, which would significantly reduce their medical applicability. Sulforaphane has a stabilising and dispersing effect, so that the conjugates of selenium nanoparticles and sulforaphane obtained have appropriate properties enabling satisfactory biodistribution of the conjugate in the bloodstream.

### Example 3 Determination of selenium content in a sample using the ICP-MS method

In order to determine the total selenium content in the sample, 1 mL of the solution was collected and subjected to microwave-assisted mineralisation in a closed circuit. This involved using 3 mL of a mixture of nitric acid (V) and perhydrol at a 3:1 ratio. A clear solution was obtained as a result of mineralization. Following an appropriate dilution of the solution, the total Se content was determined using the ICP-MS technique. The concentration was determined using a calibration curve method whose accuracy was verified by analysing a reference material having a certified selenium concentration (SPS SW1, Spectrapure Standards, Oslo, Norway). The result obtained was consistent, in terms of the uncertainty range, with the certified value. Selenium concentration in the test material was determined at 111.7 mg/L.

**Example 4 Determination of the elemental composition of the obtained conjugate through EDS analysis**

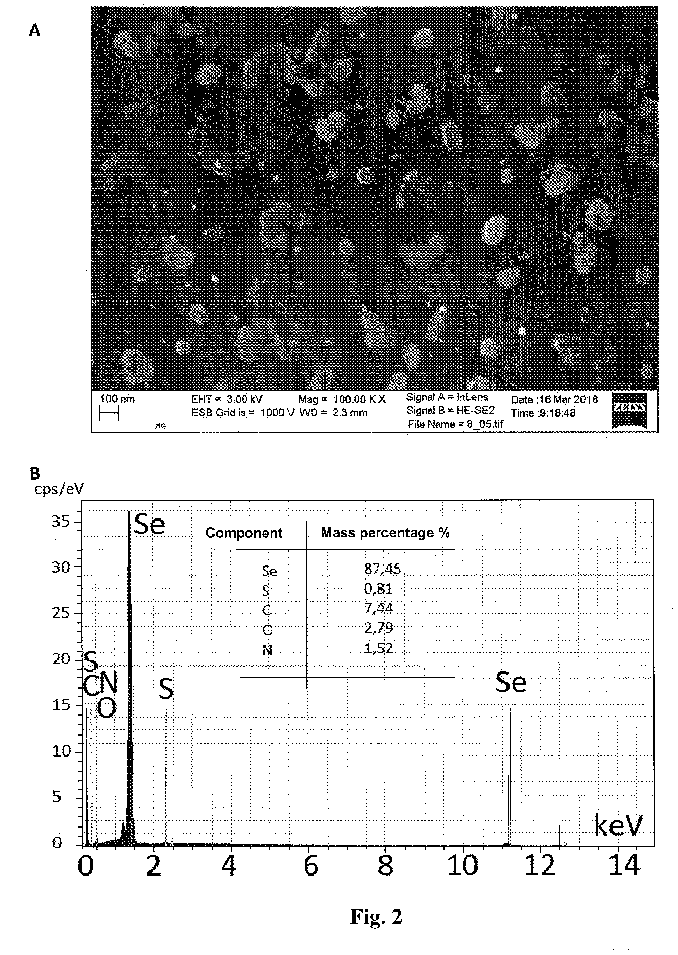

In order to obtain the test sample, 2 mL of the sample was centrifuged, and the resulting precipitate was applied onto a carbon tape placed on a metal table. **Fig. 2A** is an image of the conjugate of selenium nanoparticles and sulforaphane obtained by scanning electron microscopy (SEM). The conjugates obtained were analysed by EDS (energy-dispersive X-ray spectroscopy) in order to determine the elemental composition of the sample - **Fig. 2B.** The sample consisted of selenium Se (87.45%) and elements derived from sulforaphane, such as sulphur S (0.81%), carbon C (7.44%), oxygen O (2.79%) and nitrogen N (1.52%). The presence of sulphur indicates that sulforaphane is bound to the surface of selenium nanoparticles. The ratio of sulforaphane to selenium in the conjugate is 26 mg: 1g.

**Example 5 Determination of the elemental composition of the surface of the obtained conjugate through XPS analysis**

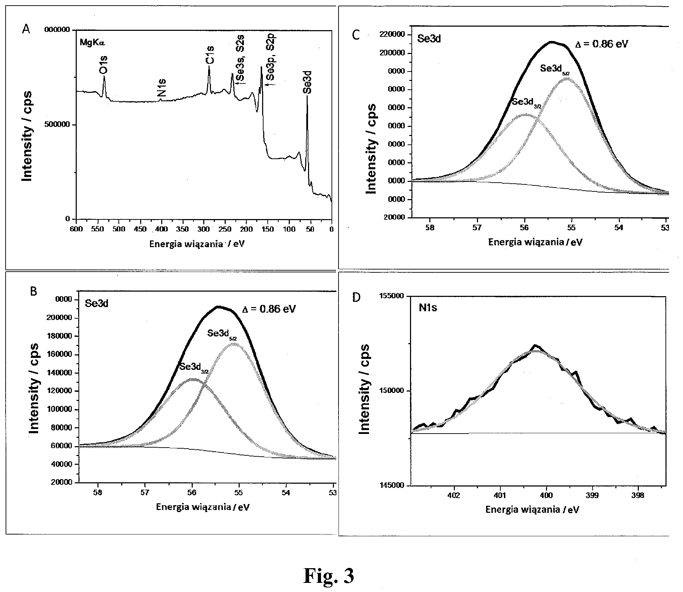

The surface of the conjugate of selenium nanoparticles and sulforaphane was analysed using the XPS (X-ray photoelectron spectroscopy) method. **Fig. 3A** presents a survey spectrum over a wide range of binding energy. Spectrum analysis confirms the presence of selenium, sulphur, nitrogen and oxygen in the sample. High resolution signal spectrum Se3d (doublet $Se3d_{3/2}$/$Se3d_{5/2}$, **Fig. 3B**) confirms the presence of elemental selenium in the sample ($Se3d_{5/2}$ signal at 55,1 eV) [J. F. Moulder et al.; Handbook of X-ray Photoelectron Spectroscopy; Perkin-Elmer Corporation 1979:92-93]. Spectrum of the S2p band (**Fig. 3C**) demonstrates the presence of two non-equivalent sulphur atoms (two doublets) derived from the sulforaphane attached to the surface of the nanoparticles. The $2p_{3/2}$ peak comprises two components with binding energy of 162.5 eV and 164.9 eV, respectively. The first component corresponds to the sulphur atom derived from the isothiocyanate group of sulforaphane [B. Folkesson et al.; An ESCA investigation of some thiocyanato complexes; J. Electron Spectrosc. Relat. Phenom. 1982:(26):157-166], while the higher energy component corresponds to the sulphur atom from the sulfoxide group of the sulforaphane [S. R. Kelemen et al.; Direct determination and quantification of sulphur forms in heavy petroleum and coals: 1. The X-ray photoelectron spectroscopy (XPS) approach; Fuel 1990:(69):939-944]. The signal shift relative to the literature value of 165.8 eV may indicate an interaction of sulforaphane with selenium nanoparticles through the oxygen atom of the sulfoxide group. The sample also contains nitrogen (N1s band at a binding energy of 400.3 eV) derived from the isothiocyanate group of sulforaphane (**Fig. 3D**). XPS analysis confirms the presence of sulforaphane on the surface of selenium nanoparticles. The determined mass ratio of sulforaphane to selenium (0.355:1) content is higher than for EDS analysis, as the XPS spectrum is collected from a maximum of 10 nm layer deep into the material.

**Example 6 Determination of the cytotoxicity of the conjugate of selenium nanoparticles and sulforaphane**

A preliminary study was carried out in order to determine the merits of using the conjugate of selenium nanoparticles and sulforaphane on breast cancer cells MDA-MB-231.

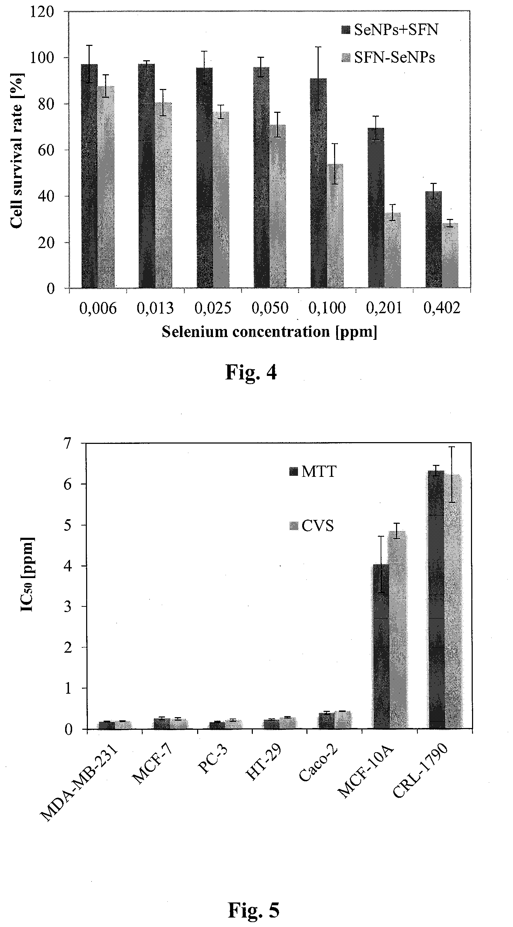

**Fig. 4** provides a comparison of the cytotoxicity of the combination of selenium nanoparticles and sulforaphane in an unbound form (SeNPs+SFN) with the conjugate of selenium nanoparticles and sulforaphane (SFN-SeNPs). A much more potent anti-tumour effect of the obtained conjugate of selenium nanoparticles and sulforaphane (SFN-SeNPs) is observed for its components administered in combination, which is most likely due to superior physical and chemical properties described in Example 2.

In order to precisely determine the cytotoxicity of the obtained conjugate of selenium nanoparticles and sulforaphane, studies were conducted of breast cancer (MDA-MB-231 and MCF-7), colon cancer (Caco-2 and HT-29), prostate cancer (PC-3) cells, and of normal colon (CRL-1790) and breast (MCF-10A) cells as control. The $IC_{50}$, parameter was determined using GraphPad Prism 5, which represents the concentration of the test compound (in this case, of the conjugate) for which the cell survival rate is 50%. **Fig. 5** features a graph presenting the $IC_{50}$ parameter value obtained through two cytotoxicity tests: MTT and CVS. The data obtained are presented in **Table 1.** The concentrations indicated are expressed as selenium content [ppm] in the sample.

*Table 1. $IC_{50}$ values and standard deviation (SD) for the conjugate of selenium nanoparticles and sulforaphane obtained using MTT and CVS assays.*

| Cell line | MTT assay | | CVS assay | |
|---|---|---|---|---|
| | $IC_{50}$ [ppm] | SD[ppm] | $IC_{50}$ [ppm] | SD[ppm] |
| MDA-MB-231 | 0.18 | 0.01 | 0.19 | 0.01 |

(continued)

| Cell line | MTT assay | | CVS assay | |
|---|---|---|---|---|
| | IC$_{50}$ [ppm] | SD[ppm] | IC$_{50}$ [ppm] | SD[ppm] |
| MCF-7 | 0.25 | 0.03 | 0.24 | 0.03 |
| PC-3 | 0.17 | 0.01 | 0.21 | 0.03 |
| HT-29 | 0.21 | 0.02 | 0.27 | 0.02 |
| Caco-2 | 0.38 | 0.04 | 0.42 | 0.01 |
| CRL-1790 | 6.31 | 0.69 | 6.22 | 0.19 |
| MCF-10A | 4.02 | 0.13 | 4.84 | 0.68 |

The obtained conjugates of selenium nanoparticles and sulforaphane are characterised by very high selectivity towards tumour cells, which means they could be of use in tumour therapy. The IC$_{50}$ parameter for normal colon CRL-1790 and breast MCF-10A cells is more than ten times higher than for breast cancer, colon cancer and prostate cancer cells. Accordingly, more than ten times less concentration is needed to achieve a cell survival rate of 50% for tumour cells compared to normal cells.

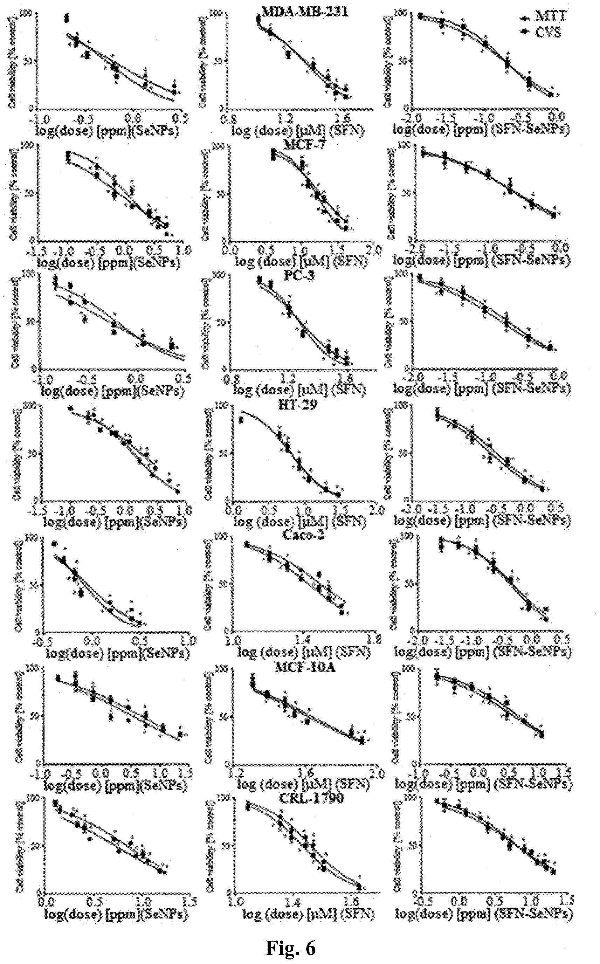

In order to determine the effect of immobilising sulforaphane on the surface of selenium nanoparticles, a study of the cytotoxicity of selenium nanoparticles, sulforaphane and conjugate of selenium nanoparticles and sulforaphane was conducted. **Fig. 6** provides a comparison of cytotoxicity of the conjugate components (selenium nanoparticles and sulforaphane) both separately and in the form of conjugate of selenium nanoparticles and sulforaphane. Modification of selenium nanoparticles by immobilising sulforaphane on their surface enhances their anti-tumour effect: the concentration of selenium is proportionately lower to obtain the same cytotoxic effect. Preferably, the toxic effect of the obtained conjugate on normal cells is reduced compared to non-modified selenium nanoparticles. This is due to the mutual interaction of sulforaphane and selenium nanoparticles, as explained in detail in Example 7.

Cell lines used:

All studied cell lines were purchased from the American Type Culture Collection (ATTC).

*MDA-MB-231 cell line*

The MDA-MB-231 cell line is a breast cancer cell line derived from a 51-year-old Caucasian woman. MDA-MB-231 cells do not express estrogen, progesterone or HER-2/Neu receptors.

*MCF-7 cell line*

The MCF-7 cell line is a breast cancer cell line derived from a 69-year-old Caucasian woman. MCF-7 cells produce estrogen, progesterone and HER-2/Neu receptors.

*PC-3 cell line*

The PC-3 cell line is a prostate cancer cell line derived from a 62-year-old Caucasian man. The cells of this line are derived from bone metastases and are highly invasive.

*HT-29 cell line*

The HT-29 cell line is a colon cancer cell line derived from a 44-year-old Caucasian woman. A large part of the HT-29 cell population are goblet cells that secrete intestinal mucus.

*Caco-2 cell line*

The Caco-2 cell line is a colon cancer cell line derived from a 72-year-old Caucasian man. These cells have absorption properties due to a system of microvilli formed on their surface.

*CRL-1790 cell line*

CRL-1790 cells are normal cells collected from the intestine of a 21-week-old female foetus. They are morphologically similar to epithelial cells.

*MCF-10A cell line*

MCF-10A cells are normal breast cells derived from a 36-year-old Caucasian woman.

*Cell culture process*

All cell lines were stored frozen in a liquid nitrogen tank at a temperature of -196°C. In order to start the culture, a vial containing the cells was heated with a stream of warm air from the dryer, after which the cell suspension was transferred to a culture bottle. The culture was carried out in the incubator in 5% atmosphere $CO_2$ and 37 °C. Cells were incubated to reach confluence of 80% and then passaged. Cells were detached using 0.25% trypsin solution in EDTA. For the CRL-1790 cell cultures, the cells were additionally scraped from the bottom of the bottle, since they did not completely detach from the medium during trypsinization. For the experiment, a suspension of MCF-7, MDA-MB-231 or PC-3 cells with a density of 40,000 cells per 1 mL of the medium was plated onto 96-well culture plates. For CRL-1790 and MCF-10A lines a cell suspension with a density of 80,000 cells per 1 mL was used, while a suspension with a density of 60,000 cells per 1 mL was prepared for Caco-2 and HT-29 cell lines. Next, a conjugate of selenium nanoparticles and sulforaphane was added to the wells in appropriate concentrations.

*CVS ( Crystal Violet Staining) test*

After washing the cell-containing wells, 50 $\mu$L 0.5% crystal violet was added to each well and incubated for 10 minutes at room temperature. The violet solution was then pipetted off and 100 $\mu$L of 1% sodium lauryl sulphate (SDS) was added to each well. The absorbance of the solution was read at $\lambda$=595 nm using the POWER WAVE XS spectrophotometer.

*MTT (methylthiazol tetrazolium salt) viability assay*

After washing, 50 $\mu$L MTT (250 $\mu$g/mL) was added to each cell-containing well, which were placed in the incubator for 3 hours. Isopropanol was then added (200 $\mu$L per well). The absorbance of the solution was read at $\lambda$=570 nm using the POWER WAVE XS spectrophotometer.

**Example 7 Determination of the interaction of selenium nanoparticles with sulforaphane**

The cytotoxicity of the conjugate of selenium nanoparticles and sulforaphane and of their components, i.e. selenium nanoparticles and sulforaphane taken separately, was studied after the incubation time of 72 hours.
Conjugates of selenium nanoparticles and sulforaphane were obtained as described in Example 1.
The tests were carried out on two cell lines of breast cancer: MCF-7 and MDA-MB-231. Control tests were performed on normal colon CRL-1790 and breast MCF-10A cells. Toxicity of the compounds i.e. how they affect the number of viable cells was determined in two cytotoxicity assays (MTT and CVS).
In order to determine the type of interaction between isothiocyanates and selenium nanoparticles, the Chou-Talaya method was used to determine the CI Combination Index) value. CI below 0.9 indicates synergy, CI between 0.9 and 1.1 indicates additive effect, while CI values above 1.1 indicate antagonism. For synergistic interactions, the effect of conjugates is more potent than that of each substance alone. In addition, combined administration allows for using smaller doses of drugs compared to individual administration.

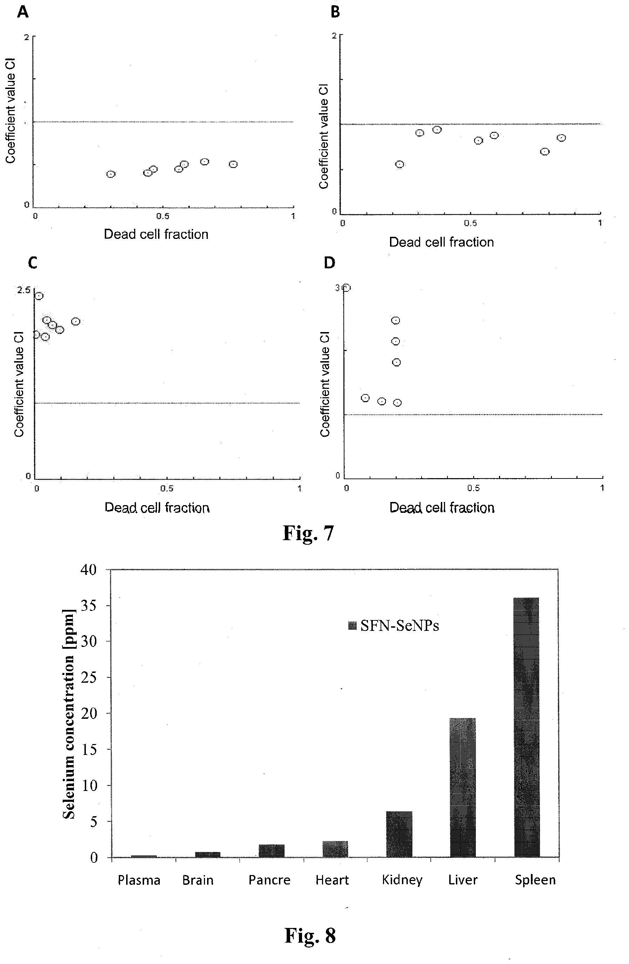

**Fig. 7** shows representative examples of testing the interaction of selenium nanoparticles and sulforaphane in *in vitro* studies towards MDA-MB-231 breast cancer cell line (A) and MCF-7 cell line (B). The conjugates of selenium nanoparticles and sulforaphane were demonstrated to have a more potent anti-tumour effect than selenium nanoparticles and sulforaphane alone, as CI values are less than 0.9 for the whole range of dead cell fraction. In addition, administration of the conjugate allows for using smaller doses of drugs compared to individual administration.
The dose reduction indices (DRI) determined indicate that it is possible to significantly reduce the dose of the individual components of the conjugate **(Table 2)**.

*Table 2.* *CI and DRI values for the conjugate of selenium nanoparticles and sulforaphane relative to MDA-MB-231 andMCF-7 cells.*

| Measuring point | MDA-MB-231 | | | | MCF-7 | | | |
|---|---|---|---|---|---|---|---|---|
| | Dead cell fraction | CI | DRI (concentration of selenium from the conjugate) | DRI (concentration of sulforaphane from the conjugate) | Dead cell fraction | CI | DRI (concentration of selenium from the conjugate) | DRI (concentration of sulforaphane from the conjugate) |
| 1 | 0.30 | 0.40 | 4.38 | 5.83 | 0.23 | 0.45 | 3.57 | 5.77 |
| 2 | 0.45 | 0.42 | 3.04 | 11.31 | 0.31 | 0.70 | 2.09 | 4.57 |
| 3 | 0.47 | 0.46 | 2.69 | 11.44 | 0.37 | 0.84 | 1.63 | 4.48 |
| 4 | 0.56 | 0.45 | 2.48 | 20.15 | 0.53 | 0.70 | 1.75 | 7.94 |
| 5 | 0.58 | 0.51 | 2.15 | 20.30 | 0.59 | 0.71 | 1.66 | 9.01 |
| 6 | 0.66 | 0.54 | 1.97 | 32.13 | 0.79 | 0.66 | 1.65 | 18.01 |
| 7 | 0.77 | 0.51 | 2.00 | 80.80 | 0.85 | 0.76 | 1.41 | 21.29 |

Tests have been performed in order to determine the type of interaction with normal CRL-1790 colon **(Fig. 7C)** and MCF-10A breast cells **(Fig.7D)** for the same concentrations of the test substances as for breast cancer cells. All experimental points had the CI value greater than 1.1, indicating antagonism. For antagonistic interactions, the effect of the conjugate is less potent than the effect of its components used separately. The use of a conjugate of selenium nanoparticles with sulforaphane allows for reducing cytotoxicity to normal cells**(Table 3)**.

*Table 3. CI values for the conjugate of selenium nanoparticles and sulforaphane to CRL-1790 and MCF-10A cells.*

| CRL-1790 | | | MCF-10A | |
|---|---|---|---|---|
| Measuring point | Dead cell fraction | CI | Dead cell fraction | CI |
| 1 | 0.01 | 2.02 | 0.01 | 2.99 |
| 2 | 0.02 | 2.29 | 0.08 | 1.26 |
| 3 | 0.04 | 1.90 | 0.15 | 1.21 |
| 4 | 0.05 | 2.12 | 0.21 | 1.18 |
| 5 | 0.07 | 1.88 | 0.21 | 1.81 |
| 6 | 0.10 | 2.17 | 0.20 | 2.14 |
| 7 | 0.16 | 2.07 | 0.20 | 2.47 |

Anti-tumour effect of selenium nanoparticles is known from prior art. Surprisingly, it has been discovered that a conjugate containing selenium nanoparticles and an isothiocyanate has improved anti-tumour properties to those of selenium nanoparticles alone, which had not been reported or suggested so far. Selenium nanoparticles modified with sulforaphane show antagonism in normal cells, which reduces cytotoxicity of the conjugate to healthy tissue, while showing synergy in tumour cells, consequently increasing the cytotoxic effect in tumour tissue. The demonstrated synergy of active substances is a highly beneficial factor, as it allows to reduce the effective concentration of the drug. Administration of the conjugate of selenium nanoparticles and sulforaphane allows for a two-fold reduction of selenium nanoparticles concentration and an eighty-fold reduction of sulforaphane concentration for the largest fractions of dead cells MDA-MB-231. Now, for the MCF-7 cell line it is possible to reduce the dose of selenium nanoparticles by half, and as regards sulforaphane, an over twenty-fold reduction is possible in the concentration of the largest fractions of dead cells. This is particularly important, as MDA-MB-231 have no estrogen, progesterone or HER-2/Neu receptors, which makes them more resistant to therapy. Indeed, conjugates of selenium nanoparticles and sulforaphane are highly cytotoxic towards said cells. Thus, the conjugate obtained is selective in terms of cytotoxicity: it is characterised by lower systemic toxicity combined with more potent anti-tumour effect, so that its properties are superior to those of selenium nanoparticles alone. Anti-tumour drugs should have properties of this kind, which means that the conjugate of to the invention will be of use in the treatment of tumour.

**Example 8 Determination of the toxicity of the conjugate of selenium nanoparticles and sulforaphane *in vivo***

The experimental procedures were carried out based on the consent no. 43/2014 issued by the IV Local Ethics Committee for Animal Experiments in Warsaw. Toxicity studies of the conjugate of selenium nanoparticles and sulforaphane were conducted on adult male rats from the Wistar Cmd:(WI)WU stock weighing 200 g. The conjugate solution and physiological saline solution (NaCl) as control were injected into the tail vein of the rat. Then, after a period of 24h, blood tests (gasometry, electrolytes, CBC and biochemical analysis) and urinalysis were performed to determine the toxicity of the conjugate. The results are presented in **Table 4.**

*Table 4. Results of gasometry, haematology, biochemical analysis and urinalysis of a control rat and a rat after administration of 100 mg of the conjugate per 1 kg body weight.*

| | Control | 100 mg/kg dose |
|---|---|---|
| **Gasometry** | | |
| pH | 7.31 | 7.36 |
| Partial pressure of carbon dioxide pCO$_2$ [mmHg] | 45 | 48 |
| Concentration of bicarbonates $HCO_3^-$ [mmol/L] | 21.0 | 25.3 |

(continued)

| | | Control | 100 mg/kg dose |
|---|---|---|---|
| **Gasometry** | | | |
| partial pressure of oxygen pO$_2$[mmHg] | | 57 | 65 |
| Sodium Na$^+$ [mmol/L] | | 136 | 141 |
| Potassium K$^+$ [mmol/L] | | 6.7 | 5.4 |
| Chlorine Cl$^-$ [mmol/L] | | 107 | 108 |
| **Haematology** | | | |
| Erythrocytes [T/L] | | 7.1 | 7.4 |
| Haemoglobin [g/dL] | | 14.7 | 15.4 |
| Hematocrit [%] | | 42.8 | 45.0 |
| MCV [fL] | | 60.2 | 60.6 |
| MCH [pg] | | 20.7 | 20.7 |
| MCHC [g/dL] | | 34.3 | 34.2 |
| Platelets [G/L] | | 834 | 987 |
| RDW-CV [%] | | 13.30 | 14.10 |
| White blood cells | Total [G/l] | 6.72 | 7.91 |
| | Neutrophils [%] | 12.5 | 32.8 |
| | Lymphocytes [%] | 82.6 | 65.4 |
| | Monocytes [%] | 3.9 | 1.5 |
| | Eosinophils [%] | 0.9 | 0.3 |
| | Basophils [%] | 0.10 | 0.00 |
| **Biochemical analysis** | | | |
| Creatinine [mg/dL] | 0.3 | 0.4 | |
| Urea [mg/dL] | 50.0 | 49.0 | |
| **Urinalysis** | | | |
| Colour | yellow | yellow | |
| Clarity | clear | clear | |
| Glucose | negative | negative | |
| Bilirubin | negative | negative | |
| Ketone bodies | negative | negative | |
| Specific weight [g/mL] | 1.025 | 1.030 | |
| Erythrocytes | negative | negative | |
| pH | 7.5 | 6.5 | |
| Protein [mg/dL] | 30 | 30 | |
| Urobilinogen [mg/dL] | 0.2 | 0.2 | |
| Nitrites | negative | negative | |
| White blood cells | trace amounts | trace amounts | |

Arterial blood gas test showed no significant difference between the control rat and the rat that has been given 100 mg of the conjugate per 1 kg of body weight. The acid-base balance, systemic gas exchange and electrolyte concentration were not disrupted by the administration of selenium nanoparticles and sulforaphane.

Neither the haematology tests nor the urinalysis showed any physiological changes in the rat that received the conjugate. All parameters are within normal limits. Protein content of 30 mg/dL in urine and trace amounts of leukocytes are a physiological phenomenon in rats [E. J. Keeble, A Meredith; BSAVA manual of rodents and ferrets; Quedgeley: British Small Animal Veterinary Association. 2009:191-302]. However, a platelet content higher in rats than in humans is also a normal phenomenon [J.E. Harkness et al.; Harkness and Wagner's Biology and Medicine of Rabbits and Rodents; 5th ed. Ames: Wiley-Blackwell. 2010: 116-131].

A biochemical test was performed in order to evaluate kidney function: blood creatinine and urea were determined. For both control and test rats, the values indicate normal kidney function.

Accumulation of selenium in rat organs was also studied. **Fig. 8** shows the concentration of selenium accumulated in rat organs obtained by the ICP-MS method. The highest concentration of selenium was found in the spleen, liver and kidneys, which is due selenium nanoparticles and sulforaphane being cleaned from the body of the rat by the reticuloendothelial system.

To conclude, the conjugate of selenium nanoparticles and sulforaphane at a concentration of 100 mg per one kilogram of body weight does not cause any toxic effects within 24 hours of administration of the preparation. Low toxicity of anti-tumour drugs is a crucial quality, which would allow the presented conjugate of selenium nanoparticles with isothiocyanates to be used in clinical therapy.

**Example 9 Determination of the pro-apoptotic effect of the conjugate of selenium nanoparticles and sulforaphane**

The mechanism of action of the conjugate selenium nanoparticles-sulforaphane was studied. MDA-MB-231 and MCF-10A cells were incubated in culture medium with or without additives. After an appropriate incubation time, measurements were made using a confocal microscope and a flow cytometer: the effect of the conjugate (SFN-SeNPs), components administered separately (SeNPs, SFN) and in combination (SFN + SeNPs) on the course of cell cycle, apoptosis induction (through detection of phosphatidylserine-V characteristic for the early stage of apoptosis) and mitochondrial membrane polarisation were determined. The cells cultured on the medium without additives were used as control.

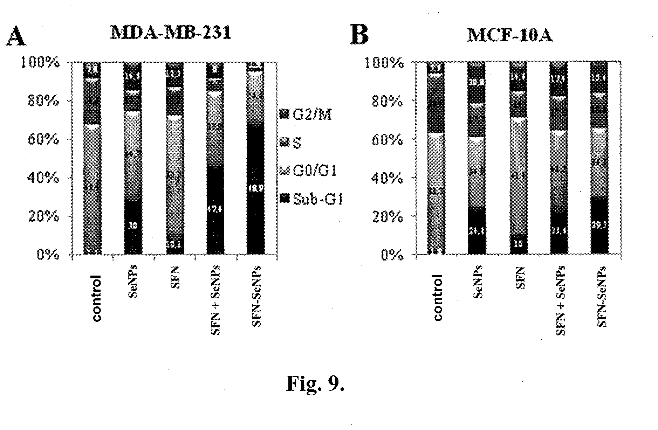

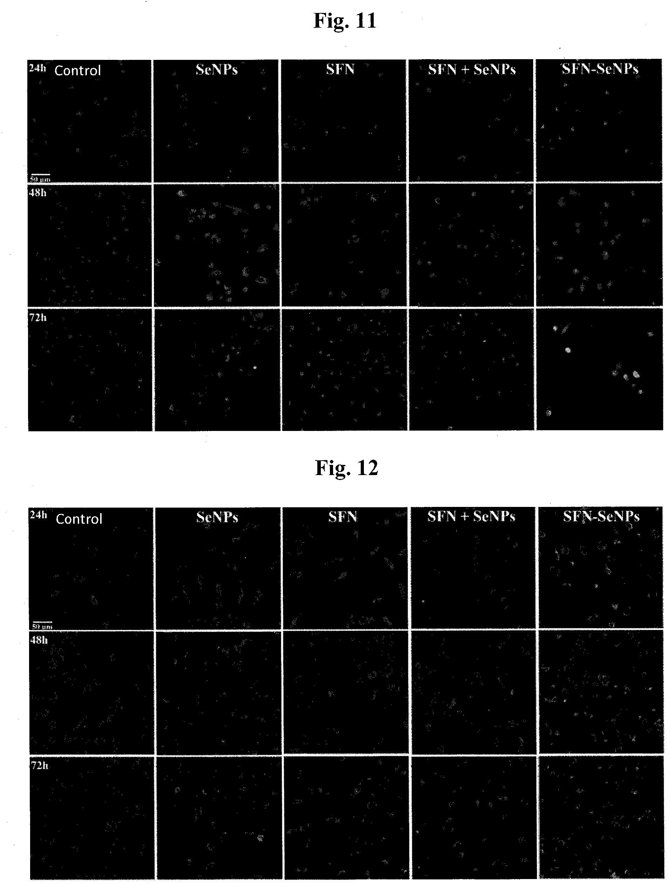

The obtained results showed that the conjugate has proapoptotic effect in cancer cells MDA-MB-231 (breast cancer). Studies of the cell cycle presented in **Fig. 9** showed substantial (almost 75% of the population), statistically significantly different from other administrations, inhibition of the cell cycle in the Sub-G1 phase, i.e. the fraction characteristic for apoptosis. Moreover, an Annexin-V externalisation study **(Fig. 10A)** showed that the vast majority of the MDA-MB-231 cancer cell population is in the early apoptosis stage. This result was confirmed microscopically (**Fig. 10B**). Microscopic studies presented in **Fig. 11** demonstrated a substantial depolarisation of the mitochondrial membrane in cancer cells, which indicates that the induction of apoptosis is mitochondrially dependent (internal pathway). **Fig. 9, Fig. 10** and **Fig. 12** show the results obtained in normal breast cells of MCF-10A. The observed changes were significantly less pronounced: cytometry showed that over 70% of the cell population are viable cells. The results obtained show that conjugate is a highly selective inductor of cell death, which results in a much more potent cytotoxic effect on tumour cells compared to normal cells. The majority of normal cells remain viable, while the majority of tumour cells die by apoptosis, which is highly desirable from the point of view of oncological therapies.

Moreover, the results obtained show that combined administration of selenium and sulforaphane increases the effect of these compounds, while the administration of a conjugate of selenium and sulforaphane has an even more potent proapoptotic effect, resulting in a more potent cytotoxic effect on tumour cells.

**Claims**

1. A conjugate having anti-tumour properties containing selenium nanoparticles modified with at least one isothiocyanate as a substance enhancing the effect of the selenium nanoparticles for use in the treatment of tumour,

   wherein the at least one isothiocyanate is sulforaphane immobilised on the surface of selenium nanoparticles, and
   wherein the tumour is breast cancer **characterised by** the absence of estrogen, progesterone and HER-2/Neu receptors.

2. The conjugate for use according to claim 1, **characterised in that** the nanoparticles of the conjugate have the size of the diameter in the range 80.2 $\pm$18.6 nm as measured with transmission electron microscopy (TEM).

**Patentansprüche**

1. Ein Konjugat mit Antitumoreigenschaften, dass Selen-Nanopartikel, modifiziert mit mindestens einem Isothiocyanat als eine die Wirkung der Selen-Nanopartikel verstärkende Substanz enthält, zur Verwendung bei der Behandlung von Tumoren, wobei das mindestens ein Isothiocyanat ein auf der Oberfläche von Selen-Nanopartikeln immobilisiertes Sulforaphan ist und wobei es sich bei dem Tumor um Brustkrebs handelt, der durch das Fehlen von Östrogen-, Progesteron- und HER-2/Neu-Rezeptoren gekennzeichnet ist.

2. Das Konjugat zur Verwendung nach Anspruch 1, **dadurch gekennzeichnet, dass** die Nanopartikel des Konjugats einen Durchmesser im Bereich von 80,2 ± 18,6 nm aufweisen, gemessen mit Transmissionselektronenmikroskopie (TEM).


**Revendications**

1. Un conjugué ayant des propriétés antitumorales contenant des nanoparticules de sélénium modifiées avec au moins un isothiocyanate en tant que la substance renforçant l'effet des nanoparticules de sélénium destiné à être utilisé dans le traitement d'une tumeur, dans lequel ledit au moins un isothiocyanate est du sulforaphane immobilisé sur la surface des nanoparticules de sélénium, et dans lequel la tumeur est un cancer du sein **caractérisé par** l'absence de récepteurs d'oestrogène, de progestérone et de HER-2/Neu.

2. Le conjugué à utiliser selon la revendication 1, **caractérisé en ce que** les nanoparticules du conjugué ont la taille du diamètre dans la plage 80,2 ± 18,6 nm tel que mesuré par microscopie électronique à transmission (TEM).

Fig. 1

**A**

| Component | Mass percentage % |
|-----------|-------------------|
| Se | 87,45 |
| S | 0,81 |
| C | 7,44 |
| O | 2,79 |
| N | 1,52 |

**Fig. 2**

**Fig. 3**

**Fig. 4**

**Fig. 5**

Fig. 6

**Fig. 7**

**Fig. 8**

Fig. 9.

Fig. 10.

**Fig. 11**

**Fig. 12**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2011010262564 A

**Non-patent literature cited in the description**

- **H. ZENG et al.** Selenium as an anticancer nutrient: roles in cell proliferation and tumour cell invasion. *International Journal of Nutritional Biochemistry,* 2008, vol. 1-7 (19
- **J. ZHANG et al.** Elemental Selenium at Nano Size (Nano-Se) as a Potential Chemopreventive Agent with Reduced Risk of Selenium Toxicity: Comparison with Se-methylselenocysteine in Mice. *Toxicol. Sci.,* 2008, vol. 22-31 (101
- **KUMARI MANISHA et al.** Curcumin loading potentiates the chemotherapeutic efficacy of selenium nanoparticles in HCT116 cells and Ehrlich's ascites carcinoma bearing mice. *European Journal of Pharmaceutics and Biopharmaceutics,* 2017, vol. 117
- **TIN KHOR et al.** Dietary Cancer Chemopreventive Agents - Targeting Inflammation and Nrf2 Signaling Pathway. *Planta Medica,* 2008, vol. 74 (13
- The Nrf2 cell defence pathway: Keap1-dependent and - independent mechanisms of regulation. **BRYAN HOLLY K et al.** Biochemical Pharmacology. Elsevier, 2012, vol. 85
- **W. LIU et al.** Selenium nanoparticles as a carrier of 5-fluorouracil to achieve anticancer synergism. *ACS Nano.,* 2012, vol. 6 (8), 6578-6591
- **J. TOMCZYK et al.** Sulforaphane - a possible agent in prevention and therapy of cancer;. *PostepyHig. Med. Dosw.,* 2010, (64), 590-603

- **Y. WANG et al.** Synergy between sulforaphane and selenium in protection against oxidative damage in colonic CCD841 cells. *Nutr. Res.,* 2015, vol. 35 (7), 610-617
- **D. LI et al.** Synergy between sulforaphane and selenium in the up-regulation of thioredoxin reductase and protection against hydrogen peroxide-induced cell death in human hepatocytes. *Food Chem.,* 2012, vol. 133 (2), 300-307
- **W. D. FOULKES et al.** Triple-negative breast cancer. *N. Engl. J. Med.,* 2010, vol. 363, 1938-1948
- **J. F. MOULDER et al.** Handbook of X-ray Photoelectron Spectroscopy. Perkin-Elmer Corporation, 1979, 92-93
- **B. FOLKESSON et al.** ESCA investigation of some thiocyanato complexes. *J. Electron Spectrosc. Relat. Phenom.,* 1982, vol. 157-166 (26
- **S. R. KELEMEN et al.** Direct determination and quantification of sulphur forms in heavy petroleum and coals: 1. The X-ray photoelectron spectroscopy (XPS) approach. *Fuel,* 1990, vol. 939-944 (69
- **E. J. KEEBLE ; A MEREDITH.** BSAVA manual of rodents and ferrets; Quedgeley. *British Small Animal Veterinary Association.,* 2009, 191-302
- **J.E. HARKNESS et al.** Harkness and Wagner's Biology and Medicine of Rabbits and Rodent. Wiley-Blackwell, 2010, 116-131